## Europäisches Patentamt
## European Patent Office
## Office européen des brevets

(11) Veröffentlichungsnummer: **0 059 701**
**B1**

(12) # EUROPÄISCHE PATENTSCHRIFT

(45) Veröffentlichungstag der Patentschrift:
10.07.85

(51) Int. Cl.⁴: **A 61 B 5/00**

(21) Anmeldenummer: 82890015.9

(22) Anmeldetag: 02.02.82

(54) Anordnung zur Messung der Wärmeabgabe und der Hauttemperatur.

(30) Priorität: 04.02.81 AT 499/81

(43) Veröffentlichungstag der Anmeldung:
08.09.82 Patentblatt 82/36

(45) Bekanntmachung des Hinweises auf die Patenterteilung:
10.07.85 Patentblatt 85/28

(84) Benannte Vertragsstaaten:
BE CH DE FR GB IT LI NL SE

(56) Entgegenhaltungen:
AT - A - 356 246
FR - A - 2 305 720
GB - A - 1 466 948
US - A - 3 339 542
US - A - 3 877 463
US - A - 4 055 166
US - A - 4 138 998

INTERNATIONAL CONFERENCE ON BIOMEDICAL TRANSDUCERS, November 1975, Seiten 109-114, Paris, FR.
NAVY TECHNICAL DISCLOSURE BULLETIN; Band 4, Nr. 3, März 1979, Seiten 53-56, Arlington, USA
MEDICAL AND BIOLOGICAL ENGINEERING, Band 14, Nr. 3, Mai 1976, Seiten 361-364, Stevenage, G.B.

(73) Patentinhaber: Innova Wiener Innovationsgesellschaft m.b.H., Beatrixgasse 1, A-1030 Wien III (AT)

(72) Erfinder: Simbruner, Georg, Dr.,
Schwarzspanierstrasse 15/215, A-1090 Wien IX (AT)

(74) Vertreter: Krause, Ernst, Dipl.-Ing. et al, Krause, Ernst, Dipl.-Ing. Casati, Wilhelm, Dipl.-Ing P.O.
Box 234 Amerlingstrasse 8, A-1061 Wien VI (AT)

**0 059 701**

**Beschreibung**

Die Erfindung betrifft eine Anordnung zur Messung der Hauttemperatur und der Wärmeabgabe einer zu untersuchenden Person.

Aus den US-PS 4 100 542, 4 055 166, 3 940 752 und 3 877 463 sind Diagnoseeinrichtungen bekannt, mit denen die Temperatur der Haut bzw. des Gewebes gemessen wird und auf Grund dieser Meßergebnisse Aussagen über den Zustand des Gewebes getroffen werden. Die Wärmeabgabe des Gewebes wird jedoch nicht zur Diagnose herangezogen.

Aus der AT-A-356 246 ist ein Ganzkörperkalorimeter bekannt, das die Wärmeabgabe eines Patienten in einer geschlossenen Kammer mißt, und zwar mit Hilfe von Temperatur- und Feuchtigkeitsmessung der in die Kammer zu- und abgeführten Luft. Als Zusatzmessung soll dabei auch die Hauttemperatur durchführbar sein. Da der Patient sich während dieser Messung in einer geschlossenen Kammer befindet, verbietet sich dabei jedwede Art von ärztlicher Behandlung.

Im menschlichen Organismus ist jedes Umsetzen von Sauerstoff eng an eine bestimmte Wärmeproduktion gekoppelt. Das Umsetzen eines ml $O_2$ erzeugt eine Wärmemenge von 19,7—21,4 J. Die entstehende Wärme ist die erste, unmittelbare Größe, welche nach dem Umsetzen des Sauerstoffes bestimmt werden kann. Im Unterschied zu vielen anderen klinischen Meßgrößen, welche das Angebot von Sauerstoff an den Körper angeben, ist die Wärmeproduktion ein Maß dafür, was tatsächlich an Sauerstoff verwertet worden ist.

Die Verteilung der Wärme im menschlichen Organismus erfolgt durch die Wärmeleitung des Gewebes (Gewebeleitung) und durch die Wärmekonvektion der Blutzirkulation. Für die Wärmeverteilung im Körper spielt die Konvektion durch die Blutzirkulation eine wesentlich größere Rolle als die Gewebeleitung; dies gilt sowohl für das absolute Ausmaß als auch für die Geschwindigkeit der Änderungen.

Ziel der Erfindung ist es, eine Anordnung zur Diagnose zu entwickeln, welche auf einem thermodynamischen Modell des menschlichen Organismus beruht und es erlaubt, aus der Wärmedynamik heraus die klinisch wichtigen Meßgrößen, wie Sauerstoffverbrauch, peripherer Blutfluß, Hautblutfluß und Wärmeleitungseigenschaft des Gewebes, Gefäßwiderstand und einige mehr zu bestimmen. Dabei soll jedoch berücksichtigt werden, daß die Belastbarkeit der zu untersuchenden Personen durch Meßgeräte begrenzt ist. Eine Vielzahl von Elektroden und Geräten werden von Erwachsenen aus psychischen und von Kindern und Neugeborenen aus geometrischen Gründen (Platzmangel) nicht zugelassen. Es sollen deshalb einige bedeutende Meßdaten ermittelt und optimal ausgewertet werden. Die Ziele werden bei einer Anordnung der eingangs genannten Art dadurch erreicht, daß

a) ein an den Rumpf der zu untersuchenden Person anlegbarer Temperaturmeßfühler und allenfalls ein Wärmeflußfühler zur Messung der Hauttemperatur an bzw. des Wärmeflusses von einer Hautstelle am Rumpf vorgesehen ist,

b) zumindest ein weiterer Temperaturmeßfühler und allenfalls ein weiterer Wärmeflußfühler vorgesehen ist, der an zumindest einem weiteren Körperteil, vorzugsweise an einem Bein oder einem Arm anlegbar ist, und der die Hauttemperatur an bzw. den Wärmefluß von der jeweiligen Hautstelle mißt,

c) bei Anordnung nur der Temperaturmeßfühler zumindest ein Meßfühler für die gleichzeitig und vorzugsweise kontinuierlich erfolgende Messung der Umgebungstemperatur vorgesehen ist, der in Verbindung mit weiteren Meßfühlern zur wirksamen Umgebungstemperatur führt,

d) aus den Signalen der Temperaturmeßfühler und dem der wirksamen Umgebungstemperatur entsprechenden Signal in einer Subtraktionsschaltung einer Auswerteinheit die Signaldifferenz zwischen den der Temperatur der jeweiligen Hautstelle des Rumpfes und der Hautstelle(n) des (der) weiteren Körperteile(s) entsprechenden Signalen und den der wirksamen Umgebungstemperatur entsprechenden Signalen ermittelbar ist, wobei die Ausgangssignalwerte dieser Subtraktionsschaltung als der jeweiligen Wärmeabgabe des Rumpfes bzw. des jeweiligen Körperteiles proportionale Werte und allenfalls die Ausgangssignalwerte der Wärmeflußfühler einer Aufzeichnungs- bzw. Anzeigeeinheit zugeführt sind.

Zur Erhöhung der Meßgenauigkeit kann für die Person ein thermisch charakterisierbarer Raum, z. B. ein kalorimeterähnlicher Behälter oder Inkubator vorgesehen sein, wobei zur Messung seiner Wand- und Außentemperatur Meßfühler vorgesehen sind, deren Meßwerte mit dem Meßwert des Meßfühlers für die Umgebungstemperatur in einer Schalteinheit zur Ermittlung eines der wirksamen Umgebungstemperatur entsprechenden Signales verknüpfbar sind.

Um sämtliche Daten jederzeit greifbar zu haben, ist es vorteilhaft, wenn die Auswerteeinheit einen Speicher für die Person betreffende Daten, z. B. Gewicht, Länge, Volumen, Oberfläche des Rumpfes, Kopfes, der Arme und Beine und Rechenkonstante, Faktoren und Proportionalitätsfaktoren aufweist, wobei die Schalteinheit der Auswerteeinrichtungen bzw. die in sie eingehenden Signalwerte mit den Speicherdaten verknüpfbar sind bzw. die einzelnen Schaltungen mit dem Speicher verbunden sind. Einfach handhabbar und aufgebaut ist die Anordnung, wenn die Temperaturmeßfühler für die Hauttemperatur und die Meßfühler für die Umgebungslufttemperatur und die Subtraktionsschaltung von heat-flux-discs bzw. Wärmeflußfühlern gebildet sind, deren Ausgangssignale an die Auswerteeinheiten geführt sind, wobei gegebenenfalls zur Messung der Hauttemperatur mindestens ein weiterer Temperaturmeßfühler vorgesehen ist.

Zur Anpassung der Meßwerte an die untersuchte Person ist vorgesehen, daß die Auswerteeinheit Korrekturschaltungen aufweist, in die gegebenenfalls vom Speicher abgerufene oder von Hand aus eingegebene, die Geometrie der Person betreffende Daten mit den den gemessenen Temperaturdifferenzen entsprechenden Signalen verknüpfbar sind, wobei über die eine Korrekturschaltung die Differenzsignale der Subtraktionsschaltung und über weitere die Korrekturschaltung die Differenzsignale der Meßfühler an die weiteren Schalteinheiten der Auswerteeinheit geführt sind.

Bei einer bevorzugten Ausführungsform der Erfindung ist vorgesehen, daß an die Ausgänge der Temperaturmeßfühler und der Meßeinrichtungen jeweils ein Multiplikator geschaltet ist, in dem die einzelnen Meßwerte jeweils für sich mit einem Faktor multiplizierbar sind, daß den Multiplikatoren jeweils ein Addierer nachgeschaltet ist, der sowohl die jeweilige berichtigte Körperkerntemperatur und Hauttemperatur des Rumpfes als auch die berichtigte Beininnentemperatur und Hauttemperatur des Beines summiert, welche Signale jeweils einer Differenzierschaltung zugeführt sind, an die eine dritte, gegebenenfalls mit dem Speicher verbundene Korrekturschaltung zur Korrektur der Werte in Abhängigkeit von der Geometrie der Person angeschlossen ist, an deren Ausgängen die zeitliche Änderungsrate der der Wärmespeicherung im Rumpf oder Bein entsprechenden Signale anliegen. Eine weitere erfindungsgemäße Ausführungsform ist dadurch gekennzeichnet, daß die Auswerteeinheit einen ersten Differenzbildner zur Differenzbildung zwischen einem der Wärmeabgabe des Beines entsprechenden Signalwert und einem ihr zugeführten oder in ihr gebildeten, einem Teil, vorzugsweise den zehnten Teil der vom Rumpf und Bein abgegebenen, gegebenenfalls im Hinblick auf gespeicherte Wärme korrigierte Gesamtwärmemenge entsprechenden Signalwert aufweist, daß an den Differenzbildner eine erste Divisionsschaltung für die Division des Wertes des Ausgangssignales des ersten Differenzbildners durch den Signalwert der in einem zweiten Differenzbildner ermittelten Differenz zwischen Körperkerntemperatur und der Beininnentemperatur angeschlossen ist, und daß das Ausgangssignal der Divisionsschaltung an die Anzeigevorrichtung als den peripheren Blutfluß vom Rumpf in das Bein proportionaler Wert geführt ist. Ferner kann erfindungsgemäß vorgeschrieben sein, daß die Auswerteeinrichtung einen zweiten Differenzbildner aufweist, in dem von den jeweils der vom Rumpf bzw. dem Bein abgegebenen Wärmemenge entsprechenden Signalwerten die der Gewebeleitung im Rumpf oder Bein entsprechenden und der Differenz zwischen Körperkerntemperatur und Hauttemperatur des Körpers bzw. Beininnentemperatur und Hauttemperatur des Beines proportionaler, in der Schalteinheit errechneten Werte abziehbar sind, welchem Differenzbildner eine weite Divisionsschaltung nachgeschaltet ist, in der die Werte des Differenzbildners durch die in einem dritten Differenzbildner gebildete Differenz der Signalwerte der Körperkerntemperatur und Hauttemperatur des Rumpfes bzw. der Beininnentemperatur und der Hauttemperatur des Beines teilbar sind und als dem jeweiligen Hautblutfluß im Rumpf und Bein proportionale Signalwerte der Auswerteeinrichtung zugeführt sind. Die erfindungsgemäße Anordnung ist durch die Verknüpfung der einzelnen Meßwerte vielfältig einsetzbar und liefert eine große Anzahl von der Diagnose dienlichen Anzeigen bzw. Ergebnissen.

Die erfindungsgemäße Anordnung wird anhand eines Ausführungsbeispieles im folgenden anhand der Zeichnung näher erläutert. Es zeigt:

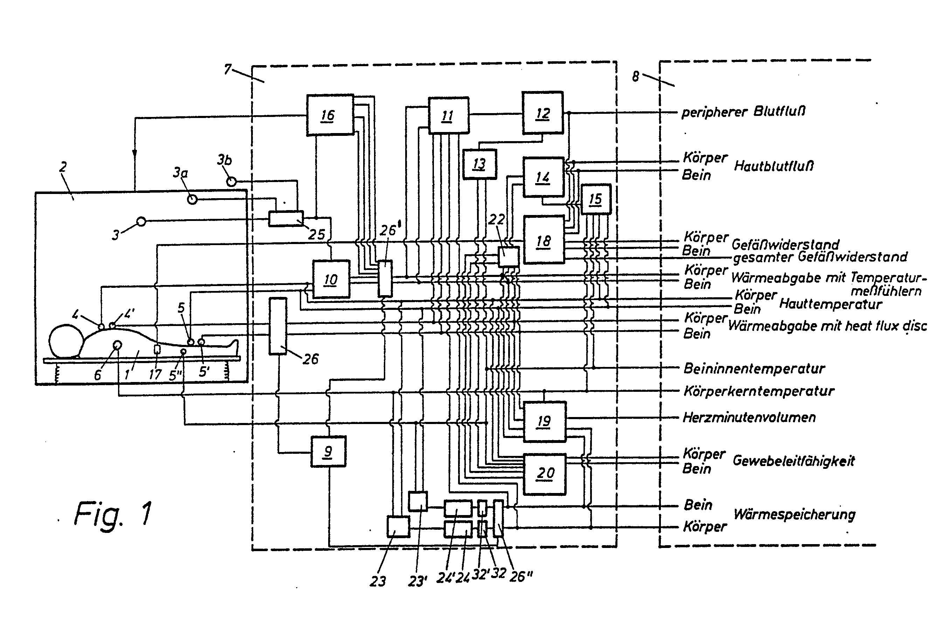

Fig. 1 ein Schaltschema der erfindungsgemäßen Anordnung bzw. Vorrichtung,

Fig. 2 ein Schema einer Temperaturmessung, und

Fig. 3 eine vereinfachte Darstellung des der Erfindung zugrundeliegenden Wärmeflußmodelles.

Die zu untersuchende Person 1 befindet sich in einem thermostabilen Raum 2, Neugeborene vorzugsweise in einem Inkubator (Brutkasten). Die Temperatur des Raumes 2 wird mit einem Meßfühler 3 und die Temperatur der Wand mit einem Meßfühler 3a gemessen. Im Sonderfall eines Inkubators kann sich ein weiterer Meßfühler 3b außerhalb des Inkubators befinden. Aus den Meßwerten dieser Meßfühler 3, 3a, 3b wird die wirksame Umgebungstemperatur errechnet.

An der minimal gekleideten Person 1 sind zwei Temperaturmeßfühler 4, 5, an besonders ausgesuchten Hautstellen angebracht. Der eine Temperaturmeßfühler 4 ist in der Mitte zwischen dem unteren Ende des Brustbeines (Xyphoid) und der linken Brustwarze (linke Mamilla) und der andere Temperaturmeßfühler 5 an einer Wade in der Mitte zwischen Fersenbein und Knie an der ventrolateralen Seite befestigt. Außerdem befindet sich ein weiterer Temperaturmeßfühler 6 entweder in der Speiseröhre in Herzhöhe oder einer bestimmten Tiefe im After, wobei die Position in der Speiseröhre vor allem beim Neugeborenen, wo der Meßfühler in Fütterungssonde integriert werden kann, und bei Schwerkranken der Vorzug gegeben wird.

Die Wahl der Stellen, an denen die Temperaturmeßfühler 4, 5, 6 angebracht werden, ist deshalb, von Bedeutung, da an den ausgewählten Stellen erfahrungsgemäß für den Rumpf bzw. das Bein charakteristische Mittelwerte der zu messenden Temperaturen herrschen.

Im beschriebenen Fall wurde der menschliche Körper in zwei Teile unterteilt. Der eine Teil umfaßt den Rumpf und den Kopf und wurde als »Rumpf« bezeichnet, der andere Teil umfaßt die Arme und Beine und wurde als »Bein« bezeichnet. Die Temperaturmessung auf der Brust wird als charakteristisch für den Rumpf mit dem Kopf, die Temperaturmessung an einem Bein als charakteristisch für alle vier Gliedmaßen angesehen.

Im folgenden werden die Messungen anhand des vorstehend beschriebenen Unterteilungsmodells des gesamten Körpers beschrieben. Es ist jedoch auch möglich, einerseits am Rumpf und andrerseits an mehreren Gliedmaßen und auch dem Kopf getrennt die Temperatur- bzw. Wärmeabgabemessungen durchzuführen; die Auswertung derartiger Messungen wird jedoch komplizierter, ohne die Genauigkeit sonderlich zu erhöhen, und ist erfahrungsgemäß nur in Spezialfällen erforderlich, da bereits durch Messung der Temperaturen am Rumpf und nur einem Bein die wesentlichen charakteristischen Meßergebnisse erhalten werden.

Unter Wärmeabgabe ist immer auch eine negative Abgabe, d. h. eine Wärmeaufnahme, zu verstehen (z. B. bei Messungen an unterkühlten Personen).

Mit der vorliegenden Vorrichtung kann auch die Wärmeverteilung im Rumpf bzw. den Armen und Beinen bestimmt werden.

Zur Ermittlung der Wärmeabgabe des Rumpfes (Kopf und Rumpf) bzw. des Beines (beide Beine und beide Arme) wird von den an den jeweiligen Hautstellen gemessenen Temperaturwerten die wirksame Umgebungstemperatur in einer Subtraktionsschaltung 10 abgezogen, so daß die einer Temperaturdifferenz entsprechenden Ausgangssignale der Subtraktionsschaltung 10 der Wärmeabgabe des Rumpfes bzw. Beines proportionale Meßwerte darstellen. Der Proportionalitätsfaktor ist bekannt, in einem Speicher 9 enthalten und wird um im Zuge der Berechnung entsprechend eingesetzt. Sodann werden in einer Korrekturschaltung 26 die Signale in Abhängigkeit von die Person 1 betreffenden Daten, wie z. B. Länge, Gewicht, Volumen usw. korrigiert.

Als Temperaturmeßfühler 4, 5 und gegebenenfalls auch als Meßfühler 3 werden vorzugsweise plattenförmige Thermistoren eingesetzt.

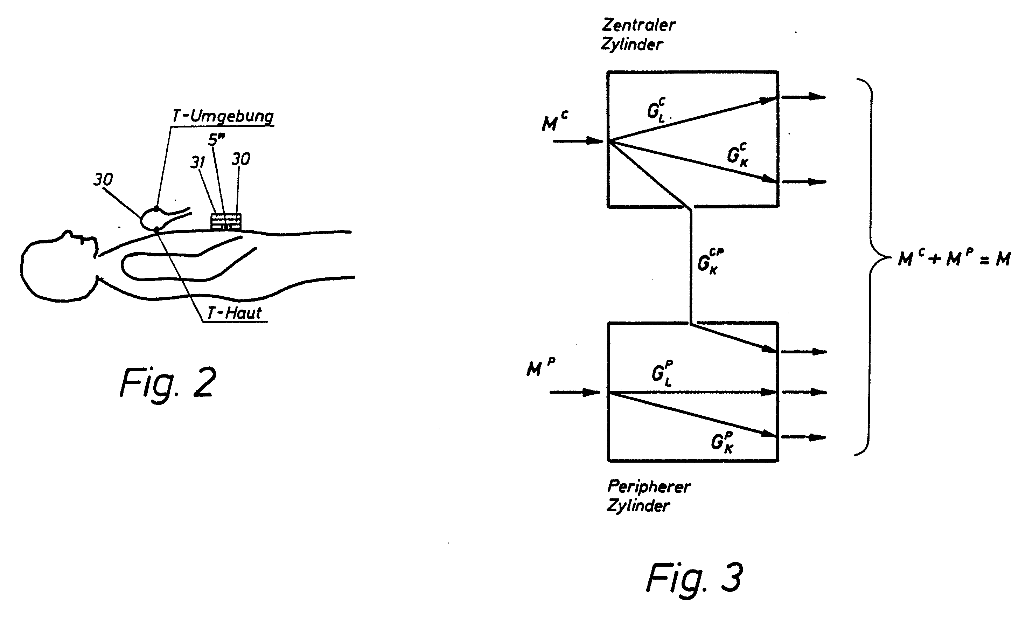

Anstelle der Temperaturmeßfühler 4, 5 und des Meßfühlers 3 für die Umgebungstemperatur und der zuvor beschriebenen Auswertung in der Subtraktionsschaltung 10 kann auch derart vorgegangen werden, daß »heat flux discs« bzw. Wärmeflußfühler 4', 5' an den zuvor bezeichneten speziellen Hautstellen eingesetzt werden. In Fig. 2 ist ein Wärmeflußfühler 30 schematisch gezeigt. Im Prinzip besteht der Wärmeflußfühler 30 aus zwei gegeneinander geschalteten Thermoelementen z. B. aus Kupfer und Konstantan, deren Lötstellen jeweils an einer Fläche der Platte angeordnet sind. Eine Plattenfläche wird mit der Haut in Berührung gebracht und nimmt Hauttemperatur an, die andere Plattenseite nimmt Umgebungstemperatur an. Die durch die Temperaturdifferenz in dem Thermokreis entstehende Spannung ist ein Maß für die vom Rumpf bzw. dem Bein abgegebene Wärmemenge. Die Signale der Wärmeflußfühler 4', 5' werden in der Auswerteeinheit 7 über die Korrekturschaltung 26' geführt; die Subtraktionsschaltung 10 ist durch die spezielle Schaltung der Thermoelemente bereits im Wärmeflußfühler selbst enthalten. Eine Korrektur dieser Meßwerte durch die Korrekturschaltung 26' erfolgt jedoch, um die Werte an die Person 1 anzupassen.

Es ist vorteilhaft, zusätzlich zu den Wärmefühlern 4', 5' noch Temperaturmeßfühler zu verwenden, jedoch nicht für die Feststellung der Wärmeabgabe, sondern für die Messung der Hauttemperatur bzw. Körperkern- oder Beininnentemperatur, wie in Fig. 2 beschrieben. In Fig. 2 ist ein Wärmeflußfühler 30 dargestellt, der einen derartigen Temperaturmeßfühler 5'' enthält und von außen mit einer plattenförmigen Heizeinrichtung 31 abgedeckt ist. Diese Anordnung dient zur Messung der Körperkerntemperatur oder der Beininnentemperatur. Dabei wird die Wärmeleistung der Heizplatte 31 so lange erhöht, bis das Ausgangssignal des Wärmeflußfühlers 30 Null ist. Zu diesem Zeitpunkt wird der Hautoberfläche vom Rumpf- bzw. Beininneren keine Wärme mehr zugeführt, da durch die Wärmezufuhr der Heizeinrichtung die Hautoberfläche auf eine Temperatur gebracht wurde, die der Körperkern- bzw. Beininnentemperatur entspricht; es besteht kein Temperaturgefälle zwischen der Hautoberfläche und dem Körperkern bzw. dem Beininneren, so daß die mit dem Temperaturmeßfühler 5'' gemessene Temperatur der Körperkerntemperatur (wenn am Rumpf gemessen wird) oder Beininnentemperatur (wenn am Bein gemessen wird) entspricht. Die Körperkerntemperatur oder Beininnentemperatur kann auch mittels einer Mikrowellensonde bzw. -antenne gemessen werden. Auch eine empirische, jedoch nicht sonderlich genaue Berechnung ist möglich, indem der jeweiligen Hauttemperatur des Rumpfes oder Beines ein Wert von vorzugsweise 0,8° C hinzugefügt wird.

Zur Speicherung von Parametern, Konstanten und Rechenfaktoren, die bekannt sind oder sich im Zuge der Berechnung ergeben und weiterverwendet werden, und von Daten betreffend die zu untersuchende Person, wie z. B. Länge, Gewicht, Körperoberfläche, Volumen des Körpers, der Gliedmaßen usw. ist in der Auswerteeinrichtung 7 der Speicher 9 vorgesehen, der nach Bedarf mit den einzelnen, vorzugsweise allen Rechenstufen der Auswerteeinheit 7 entsprechend verbunden ist, und von ihm sind bei Bedarf entsprechende Werte abrufbar. Der Speicher 9 ergänzt die Korrekturschaltung 26 bzw. 26' bzw. 26'', die mit von Hand aus eingegebenen oder vom Speicher abgerufenen Werten, die Wärmeabgabesignale bzw. die entsprechenden Temperaturen bzw. Temperaturdifferenzen unter Berücksichtigung der Form bzw. Abmessungen der Person 1 anpaßt. Mit den Meßeinrichtungen werden den tatsächlichen Werten der Person proportionale Werte ermittelt, die bei entsprechender Eichung der Vorrichtung und entsprechender Verknüpfung mit den Daten des Speichers 9 bzw. Daten der Korrekturschaltung 26 die absoluten Werte der zu messenden Größen darstellen. Die erfindungsgemäße Vorrichtung kann als analog oder digital rechnende Vorrichtung aufgebaut sein; bevorzugt ist es, wenn ein digitaler Rechner eingesetzt wird, wozu eine entsprechende Analog-Digital-Umsetzung der Meßwerte an entsprechenden Stellen vorgesehen ist. Die Auswerteeinheit 7 enthält entsprechende Rechenelemente, welche die beschriebenen Rechenoperationen ausführen. An die Auswerteeinheit 7 schließt eine Registrier- oder Anzeigeeinrichtung 8 an, an der die einzelnen gemessenen und berechneten Werte aufscheinen bzw. ablesbar sind. Es ist verständlich, daß die erhaltenen Werte auch weiterverarbeitet werden können.

Durch die Messung der Wärmeabgabe des Rumpfes und des Beines werden Rückschlüsse auf die Wärmeproduktion des Rumpfes (Rumpf und Kopf) und des Beines (beide Arme und Beine) bzw. durch die Messung der Kerntemperaturen Rückschlüsse auf die Wärmeverteilung gezogen. Da die Wärme bzw. -produktion direkt proportional dem Sauerstoffverbrauch ist, kann durch Beobachtung der Wärmeabgabe der Verbrauch an Sauerstoff bzw. die chemische Oxidation der Nahrung verfolgt werden. Es kann eine Energiebilanz erstellt werden, da die Energiezufuhr gleich ist dem Energieverlust durch die Wärmeabgabe (und der geleisteten Arbeit + Evaporation + insensibler Wasserverlust) zuzüglich der Energiespeicherung im Körper (Gewebeaufbau + Wärmespeicherung).

Da bei zu niedriger und zu hoher wirksamer Umgebungstemperatur die Wärmeabgabe aufgrund vermehrten Energiebedarfs (Sauerstoffverbrennung) ansteigt, ist eine Regeleinrichtung 16 vorgesehen, mit der in Abhängigkeit von der Wärmeabgabe, vorzugsweise der Summe der Wärmeabgabe von Rumpf und Bein, die Umgebungstemperatur des thermisch stabilen Raumes 2 auf einen optimalen Wert eingeregelt werden kann, bei dem die Wärmeabgabe ein Minimum erreicht hat. Der Regler 16 ist hierzu an die Meßfühler 3, 3a, 3b und bzw. die Subtraktionsschaltung 10 gegebenenfalls an die Korrekturschaltung 26 und/oder an die Wärmeflußfühler 4', 5' angeschlossen. Bei der Messung der Umgebungstemperatur mit den Meßfühlern 3, 3a und 3b ist zu berücksichtigen, daß der Körper und das Bein durch Abstrahlung und Konvektion Wärme verlieren. Die operante bzw. wirksame Umgebungstemperatur wird demzufolge berechnet bzw. werden die Meßwerte des Meßfühlers 3 einer Subtrahier- bzw. Multiplizier- bzw. Addierschaltung 25 zugeführt, in der die Differenz der Meßwerte und der Wand- oder Außentemperatur gebildet und diese Differenz mit einem Faktor, vorzugsweise mit 1/7 multipliziert wird. Der resultierende Wert wird als Korrekturwert von der mit dem Meßfühler 3 gemessenen Lufttemperatur abgezogen und als Wert für die wirksame Umgebungstemperatur der Auswerteeinheit 7 bzw. Korrekturschaltung 26 zur Verfügung gestellt. Es ist auch möglich, die wirksame Umgebungstemperatur zu berechnen, indem in der Schalteinheit die Wandtemperatur mit einem Faktor, vorzugsweise 0,6, und die Lufttemperatur mit einem Faktor, vorzugsweise 0,4, multipliziert werden und sodann die Werte summiert werden.

Zur Berechnung des peripheren Blutflusses, d. h. des Blutflusses vom Rumpf in die Beine und Arme, wird folgendermaßen vorgegangen:

Die vom Bein abgegebene Wärmemenge entspricht dem dem Bein durch die Blutzirkulation zugeführten Wärmefluß zuzüglich der im Bein produzierten Wärme. Die zirkulierte Wärme bzw. der Wärmefluß ins Bein ist dem Produkt aus dem peripheren Blutfluß und der Temperaturdifferenz zwischen arteriellem und venösem Blut proportional, die wiederum der Temperaturdifferenz zwischen dem Körperkern und dem Beininneren proportional ist. Der letztere Proportionalitätsfaktor beträgt vorzugsweise 0,25. Wenn man die vom Bein produzierte Wärmemenge als vorzugsweise 1/10 der gesamten von Rumpf und Bein abgegebenen Wärmemenge setzt und die Körperkerntemperatur bzw. die Beininnentemperatur entweder mittels Mikrowellenantenne oder auf die in Fig. 2 gezeigte Art mißt oder als 0,8° C über der Hauttemperatur liegend festsetzt, so kann der periphere Blutfluß bestimmt werden. Der Faktor 0,25, der Wert 0,8° C und der Wert 1/10, die spezifische Wärme des Blutes usw. sind im Speicher 9 enthalten und zu gegebener Zeit den Recheneinheiten zuführbar.

In der Auswerteeinheit 7 ist hierfür eine erste Divisionsschaltung 12 vorgesehen, deren einem Eingang ein Differenzbildner 13 für die Werte der Körperkerntemperatur (vom Meßfühler 6) und die Beininnentemperatur (gemessen z. B. auf die in Fig. 2 dargestellte Art) vorgeschaltet ist. Dem anderen Eingang ist ein Differenzbildner 11 vorgeschaltet, dem die Werte der Wärmeabgabe von Rumpf und Bein zugeführt sind. Die Werte der Wärmeabgabe können mit Werten für die im Körper oder Bein gespeicherte Wärme aus später beschriebenen Addierschaltungen 24, 24' korrigiert werden. Der Differenzbildner 11 berechnet unter Verwendung des im Speicher 9 gespeicherten Proportionalitätsfaktors (1/10) für das Verhältnis der vom Bein produzierten Wärmemenge zu der gesamten von Rumpf und Bein abgegebenen Wärmemenge die Differenz zwischen der vom Bein abgegebenen und produzierten Wärmemenge. Diese Differenz entspricht der dem Bein durch die Blutzirkulation zugeführten Wärmemenge. In der Divisionsschaltung 12 wird das vom Differenzbildner 11 zugeführte Wärmedifferenzsignal durch die Temperaturdifferenz des Differenzbildners 13 dividiert, und am Ausgang der Divisionsschaltung liegt der Wert des peripheren Blutflusses an.

Zur Berechnung des Hautblutflusses bzw. des Blutflusses vom Rumpf- bzw. Beininneren zur jeweiligen Hautoberfläche wird von der vom Rumpf oder Bein abgegebenen Wärmemenge die durch die Gewebeleitung transportierte Wärme abgezogen. Diese Wärmemenge wird aus der Temperaturdifferenz zwischen dem Körperkern- bzw. Beininneren und den jeweiligen Hautoberflächen in der Schalteinheit 20 errechnet. Einer zweiten Divisionsschaltung 14 ist ein dritter Differenzbildner 15 vorgeschaltet, in dem die Differenz zwischen der Körperkern- bzw. Beininnentemperatur und den jeweiligen Hauttemperaturen gebildet wird. In der zweiten Divisionsschaltung 14 werden die um die Werte der Gewebswärmeleitung verminderten Werte der Wärmeabgabe durch die Temperaturdifferenzen geteilt. Am Ausgang der zweiten Divisionsschaltung 14 liegen die dem Hautblutfluß im Körper oder im Bein entsprechenden Werte an.

Da der Gefäßwiderstand dem Verhältnis von Blutdruck und Blutfluß proportional ist, kann bei Bekanntsein des Wertes des Blutflusses und des Blutdruckes ein Wert für den Gefäßwiderstand erhalten werden. Dazu werden die Signale eines Blutdruckmeßgerätes 17 einer dritten Divisionsschaltung 18 zugeführt, an dessen anderem Eingang die dem peripheren Blutfluß bzw. dem Hautblutfluß im Rumpf oder Bein entsprechenden Signale anliegen. Am Ausgang der dritten Divisionsschaltung 18 liegen die dem jeweiligen Gefäßwiderstand proportionalen oder direkt entsprechenden Werte an.

Der von der Person 1 verbrauchte Sauerstoff ist dem Pumpvolumen des Herzens und der Sauerstoffdifferenz arteriellen und venösen Blut proportional. Bekannterweise ist diese Sauerstoffdifferenz der Körperkerntemperatur proportional; der verbrauchte Sauerstoff ist, wie eingangs erwähnt, der gesamten abgegebenen und gespeicherten Wärmemenge proportional. Es ist eine vierte Divisionsschaltung 19 vorgesehen, in der die gesamte von Rumpf und Bein abgegebene Wärmemenge, gegebenenfalls zuzüglich einer Wärmemenge entsprechend den Signalen der Addierer 24, 24' durch die Körperkerntemperatur teilbar ist. Am Ausgang der vierten Divisionsschaltung 19 liegt ein dem Herzminutenvolumen entsprechender Wert an. Es ist auch möglich, der Auswerteeinheit 7 Differenzierglieder hinzuzufügen, denen die Ausgangssignale der einzelnen Schalteinheiten zugeführt sind, um die zeitliche Änderung der Werte verfolgen zu können bzw. Werte für die Signaländerung an sich zu erhalten.

Zur Ermittlung der vom Körper oder dem Bein gespeicherten Wärme wird die Körperkerntemperatur bzw. Beininnentemperatur jeweils in Multiplikatoren 23, 23' mit einem Faktor, vorzugsweise 0,6, multipliziert. In den Multiplikatoren 23, 23' wird ferner die Hauttemperatur des Rumpfes bzw. Beines jeweils mit einem Faktor, vorzugsweise 0,4 multipliziert. In nachfolgenden Addierern 24, 24' wird die Summe der korrigierten Körperkerntemperatur und Hauttemperatur des Rumpfes bzw. der Beininnentemperatur und der Hauttemperatur des Beines gebildet. Aus diesen Werten werden in nachfolgenden Differenziergliedern 32, 32' die zeitlichen Änderungsraten ermittelt; diese Werte werden in einer an den Speicher 9 angeschlossenen Korrekturschaltung 26'', unter Berücksichtigung der Geometrie des Körpers und entsprechender physikalischer Konstanten korrigiert und gelten als Maß für den zeitlichen Wärmeverlust oder die zeitliche Wärmespeicherung im Rumpf oder Bein. Diese Werte werden auch der vierten Divisionsschaltung 19 bzw. dem ersten Differenzbildner 11 zugeführt, um bei dynamischen Messungen den Meßwert für die abgegebene Wärme, der weiterverarbeitet wird, vor der Weiterverarbeitung entsprechend korrigieren. Diese Meßmethode wird insbesondere dann angewandt, wenn eine dynamische Untersuchung vorgenommen wird und Änderungen der Wärmemengen von Bedeutung sind.

Im folgenden wird die Erfindung noch genauer unter Bezugnahme auf die verwendeten Formeln und Näherungen bzw. gewählten Proportionalitätsfaktoren erläutert:

Die Geometrie der Person wurde durch Zylinder approximiert; es wurden zwei Zylinder (Körper, Bein) eingeführt, so daß der periphere Blutfluß errechnet werden kann. Rumpf und Kopf wurden durch einen Zylinder (Körper bzw. zentraler Zylinder) und die Extremitäten durch einen zweiten Zylinder (Bein bzw. peripherer Zylinder) dargestellt.

Der Wärmefluß innerhalb jedes Zylinders und zwischen den beiden Zylindern ist in Fig. 3 dargestellt. Die Wärmemenge im zentralen Zylinder, welche der Metabolismus produziert, wird a) durch Konduktion und Konvektion an seine Oberfläche und b) durch Konvektion mittels des peripheren Blutflusses in den peripheren Zylinder transportiert. Die Wärmemenge im peripheren Zylinder, welche teils vom Metabolismus produziert und teils vom zentralen Zylinder importiert wird, wird ebenso durch Konduktion und Konvektion an die Oberfläche des peripheren Zylinders transportiert. Der Wärmeverlust von der Oberfläche jedes Zylinders erfolgt durch Konvektion und Radiation. Die Meßgrößen, welche für die Eingabe notwendig sind und welche für die Ausgabe errechnet werden, sind in Tabelle 1 zusammengefaßt. Nur einige Eingabegrößen müssen direkt gemessen werden, die übrigen können von Tabellen abgelesen werden.

Tabelle 1
Ein- und Ausgabegrößen des thermodynamischen Modelles

Eingabe

| | | |
|---|---|---|
| $V$ | Körpergewicht i. e. Körpervolumen (ml) | |
| *) $V_A, V_B$ | Volumen des Armes, Beines (ml) | |
| $L$ | Körperlänge (cm) | |
| *) $L_A, L_B$ | Länge des Armes, Beines (cm) | |
| *) $A$ | Gesamte Körperoberfläche (cm$^2$) | |
| $T_a$ | Temperatur der Inkubatorluft (°C) | |
| **) $T_W$ | Temperatur der Inkubatorwand (°C) | |
| **) $T_r$ | Raumtemperatur (°C) | |
| $T_{cc}$ | zentrale Kerntemperatur (°C) | |
| $T_{cs}$ | zentrale Hauttemperatur (°C) | |
| $T_{ps}$ | periphere Hauttemperatur (°C) | |

Ausgabe

| | |
|---|---|
| M | Gesamte Wärmeproduktion (Watt oder Watt/kg) |
| $\dot{V}O_2$ | Sauerstoffverbrauch (ml/min oder ml/min.kg) |
| $M_{cp}$ | Peripherer Wärmefluß (Watt oder Watt/100 ml Gewebe des zentralen oder peripheren Zylinders) |
| $\dot{V}_{cp}$ | Peripherer Blutfluß (ml/min. 100 ml Gewebe des zentralen oder peripheren Zylinders) |

*) Alle diese Größen können aus einem Nomogramm abgelesen werden.
**) Nur eine von den zwei Größen muß gemessen werden.

Die Bezeichnungen in Fig. 3 bedeuten:
$M^c$ = Metabolismus im zentralen Zylinder, $M^p$ = Metabolismus im peripheren Zylinder, $G_L$ = Wärmekonduktion des Gewebes, $G_K$ = Wärmekonvektion durch den Blutfluß.

Von den Eingabegrößen (Körpergewicht und -länge) werden die Volumina und Oberflächen der Zylinder abgeleitet. Das Körpergewicht wird dem Körpervolumen gleichgesetzt, indem ein spezifisches Gewicht von 1 g/cm³ angenommen wird. Das Volumen des zentralen Zylinders entspricht dann dem Körpervolumen minus dem Extremitätenvolumen (2 Arm- und 2 Beinvolumina). Die Oberfläche der beiden Zylinder erhält man in zwei Schritten. Im ersten Schritt werden die Mantelflächen des zentralen und peripheren Zylinders aus deren Volumina und Längen ermittelt (Gleichungen 1a und 1b):

$$\bar{A}_c = 2\sqrt{V_c(L-L_B)\pi}, \tag{1a}$$

$$\bar{A}_p = 2\sqrt{V_p \cdot 2(L_A-L_B)\pi}. \tag{1b}$$

In einem zweiten Schritt werden dann die für den Wärmeverlust effektiven Oberflächen errechnet. Die gesamte Körperoberfläche, aus einem Nomogramm abgelesen, wird entsprechend dem Verhältnis der beiden Zylindermantelflächen aufgeteilt, und der Proportionalitätsfaktor q ($q = \bar{A}_p/\bar{A}_c + \bar{A}_p$) ergibt die Oberfläche ($A_p$) des peripheren Zylinders (siehe Gleichung 2b). Von der gesamten Körperoberfläche wird daraufhin die Oberfläche des peripheren Zylinders ($A_p$) und des Rückens ($A_b$ = 9% der gesamten Körperoberfläche) abgezogen, und man erhält so die Oberfläche des zentralen Zylinders ($A_c$) (Gleichung 2b):

$$A_c = (1-b-q) \cdot A, \tag{2a}$$

$$A_p = q \cdot A. \tag{2b}$$

Diese Berechungen erfolgen soweit als möglich in der Korrekturschaltung 26, gegebenenfalls in Verbindung mit dem Speicher 9.

Unter Gleichgewichtsbedingungen ist die Wärmeproduktion gleich dem Wärmeverlust. Der Wärmeaustausch zwischen dem Organismus und der Umgebung kann durch die Gleichung 3 beschrieben werden:

$$M-W = h_r \cdot A(T_s-T_w) + h_c \cdot A(T_s-T_a) + E, \tag{3}$$

und wenn man die Arbeit (W) und die Evaporation (E) vernachlässigt, erhält man die Gleichung 4:

$$M = h_r \cdot A(T_s-T_w) + h_c \cdot A(T_s-T_a). \tag{4}$$

Diese Gleichung besagt, daß der Wärmeaustausch durch Radiation und Konvektion zustande kommt. Radiation und Konvektion sind proportional der Oberfläche und der Temperaturdifferenz zwischen mittlerer Hauttemperatur ($T_s$) (gemessen mit den Temperaturmeßfühlern 4, 5, 5') und der Temperatur der Wand ($T_w$) bzw. der Luft ($T_a$) (gemessen mit Meßfühler 3a bzw. 3). $h_r$ und $h_c$ sind die Wärmeleitungskoeffizienten. Formt man die Gleichung 4 um, indem man die Klammern auflöst und die zwei Glieder des Polynoms, $h_r A T_w$ und $h_c A T_a$, durch $h_r + h_c/(h_r + h_c)$ erweitert, erhält man die Gleichung 5:

$$M = A(h_r + h_c) \cdot (T_s - (h_r T_w + h_c T_a)/(h_r + h_c)). \tag{5}$$

Wenn ein kombinierter Wärmeleitungskoeffizient h ($h = h_r + h_c$) und eine operante Umgebungstemperatur $T_o$ ($T_o = (h_r T_w + h_c T_a)/(h_r + h_c)$) eingeführt wird, erhält man die vereinfachte Gleichung 6:

$$M = A \cdot h(T_s - T_o). \tag{6}$$

Diese Berechung erfolgt in der Schalteinheit 25, der Korrekturschaltung 26 und der Subtraktionsschaltung 10 (oder durch die Verwendung von Wärmeflußfühlern). Der kombinierte Wärmeleitungskoeffizient h wurde entsprechend den Angaben mit $6,973 \cdot 10^{-4} W/cm^2 \cdot °C$ festgelegt. Die operante Umgebungstemperatur wurde durch zwei Näherungsformeln, Gleichung 7a und 7b approximiert (in der Schalteinheit 25):

$$T_o = 0,6\, T_w + 0,4\, T_a, \qquad (7a)$$

$$T_o = T_a - (T_a - T_r)/7. \qquad (7b)$$

Wenn nun die Gleichung 6 auf das Zwei-Zylinder Modell angewendet wird, erhält man die Gleichung 8:

$$M = M_c + M_p = h\,(A_c(T_{cs} - T_o) + A_p\,(T_{ps} - T_o)). \qquad (8)$$

Diese Gleichung enthält den kombinierten Wärmeleitungskoeffizienten h, die für den Wärmeaustausch wirksamen Oberflächen (siehe Gleichungen 2a und 2b), die operante Umgebungstemperatur und die mittlere Hauttemperatur des zentralen ($T_{cs}$) und peripheren ($T_{os}$) Zylinders.

Die mittlere Hauttemperatur des zentralen Zylinders wird durch die thorakale Hauttemperatur zwischen Xyphoid und linker Mamilla und die mittlere Hauttemperatur des peripheren Zylinders durch die Waden-Hauttemperatur zwischen Knie und Ferse repräsentiert. Eine Grundsatzstudie hatte gezeigt, daß diese beiden Temperaturen eine gute Annäherung darstellen. Löst man die Gleichung 8 getrennt bzw. durch Subtraktion der Temperaturen, erhält man die gesamte Wärmemenge, welche vom Neugeborenen produziert (und abgegeben) wird. Diese Wärmeproduktion wird in einen Sauerstoffverbrauch umgewandelt, indem man annimmt, daß jeder ml Sauerstoff, der verbrannt wird, etwa 5 cal liefert. Einer thermochemischen Kalorie entsprechen 4,1840 Joule, einer Kalorie/min 4,1840 Joule/60 bzw. 0,06973 Watt, und folglich entsprechen 1 ml Sauerstoffverbrauch/min $5 \cdot 0,06973$ Watt. Daher gilt Gleichung 9:

$$\text{Sauerstoffverbrauch } \dot{V}O_2\ ml/min = M\ (Watt)/5 \cdot 0,06973. \qquad (9)$$

Bei der Kalkulation des peripheren Blutflusses ($V_{cp}$) wird angenommen, daß der Metabolismus des peripheren Zylinders die Wärmemenge $M_p$ produziert, und es wurde angenommen, daß $M_p$ ein Zehntel des Gesamtmetabolismus ausmacht (Gleichung 10):

$$M_p = \varkappa \cdot M \qquad (10)$$

wobei $\varkappa$ 1/10 ist.

Der periphere Blutfluß bringt die Wärmemenge $M_{cp}$ vom zentralen in den peripheren Zylinder. Die vom Metabolismus produzierte Wärmemenge $M_p$ und die vom peripheren Blutfluß importierte Wärmemenge $M_{cp}$ wird an der Oberfläche des peripheren Zylinders abgegeben, entspricht also dem Wärmeverlust von dessen Oberfläche an die Umgebung, daher gilt Gleichung 11:

$$\varkappa M + M_{cp} = h \cdot A_p\,(T_{ps} - T_o)\ bzw.$$
$$M_{cp} = h \cdot A_p\,(T_{ps} - T_{or})\,\varkappa M. \qquad (11)$$

Löst man die Gleichung 11, erhält man die Wärmemenge $M_{cp}$, welche der Blutfluß $\dot{V}_{cp}$ vom zentralen in den peripheren Zylinder transportiert. Die Wärmemenge $M_{cp}$ in Watt ergibt entsprechend der zuvor festgelegten Proportionalität, 1 Kalorie/min = 0,06973 Watt, bzw. 1 Joule/min = 0,01674 Watt, die Wärmemenge $\dot{Q}_{cp}$ in cal/min bzw. Joule/min. Diese konvektierte Wärme ist proportional der spezifischen Wärme des Blutes, der Blutflußmenge und der Temperaturdifferenz zwischen arteriellem ($T_{art}$) und venösem ($T_{ven}$) Blut. Diesen Zusammenhang beschreibt die Gleichung 12:

$$\dot{Q}_{cp} = c_{bl} \cdot \dot{V}_{cp}\,(T_{art} - T_{ven}). \qquad (12)$$

Die spezifische Wärme des Blutes $c_{bl}$ wurde mit 0,92 cal/ml.°C angenommen. Außerdem wurde festgelegt, daß die Bluttemperaturdifferenz der Differenz der Kerntemperaturen von zentralem und peripherem Zylinder proportional ist, daher gilt Gleichung 13:

$$T_{art} - T_{ven} = \alpha\delta\,(T_{cc} - T_{pc}). \qquad (13)$$

Als Proportionalitätsfaktor $\alpha$ wurde auf Grund tierexperimenteller Studien vorzugsweise 0,25 eingeführt. Die Kerntemperatur des zentralen und peripheren Zylinders wird wie beschrieben gemessen. Wenn die Gleichung 12 nach $\dot{V}_{cp}$ aufgelöst und für $\dot{Q}_{cp}$ und die arterio-venöse Temperaturdifferenz eingesetzt wird, erhält man die Gleichung 14 für den peripheren Blutfluß

$$\text{Peripherer Blutfluß } \dot{V}_{cp}\ ml/min = M_{cp}\ Watt/0,92 \cdot 0,25 \cdot 0,06973\,(T_{cc} - (T_{ps} + 0,5)). \qquad (14)$$

**0 059 701**

## Patentansprüche

1. Anordnung zur Messung der Wärmeabgabe und der Hauttemperatur einer zu untersuchenden Person, dadurch gekennzeichnet, daß

a) ein an den Rumpf der zu untersuchenden Person (1) anlegbarer Temperaturmeßfühler (4) und allenfalls ein Wärmeflußfühler (4') zur Messung der Hauttemperatur an bzw. des Wärmeflusses von einer Hautstelle am Rumpf vorgesehen ist,

b) zumindest ein weiterer Temperaturmeßfühler (5) und allenfalls ein weiterer Wärmeflußfühler (5') vorgesehen ist, der an zumindest einem weiteren Körperteil, vorzugsweise an einem Bein oder einem Arm anlegbar ist, und der die Hauttemperatur an bzw. den Wärmefluß von der jeweiligen Hautstelle mißt,

c) bei Anordnung nur der Temperaturmeßfühler (4, 5) zumindest ein Meßfühler (3) für die gleichzeitig und vorzugsweise kontinuierlich erfolgende Messung der Umgebungstemperatur vorgesehen ist, der in Verbindung mit weiteren Meßfühlern (3a, 3b) zur wirksamen Umgebungstemperatur führt,

d) aus den Signalen der Temperaturmeßfühler (4, 5) und dem der wirksamen Umgebungstemperatur entsprechenden Signal in einer Subtraktionsschaltung (10) einer Auswerteinheit (7) die Signaldifferenz zwischen den der Temperatur der jeweiligen Hautstelle des Rumpfes und der Hautstelle(n) des (der) weiteren Körperteile(s) entsprechenden Signalen und den der wirksamen Umgebungstemperatur entsprechenden Signalen ermittelbar ist, wobei die Ausgangssignalwerte dieser Subtraktionsschaltung (10) als der jeweiligen Wärmeabgabe des Rumpfes bzw. des jeweiligen Körperteiles proportionale Werte und allenfalls die Ausgangssignalwerte der Wärmeflußfühler (4', 5') einer Aufzeichungs- bzw. Anzeigeeinheit (8) zugeführt sind.

2. Anordnung nach Anspruch 1, dadurch gekennzeichnet, daß ein weiterer Temperaturmeßfühler (6) zur Messung der Körperkerntemperatur vorgesehen ist, der z. B. in die Speiseröhre, in die Höhe des Herzens oder in den After einführbar ist, und daß sein Ausgangssignal der Auswerteeinheit (7) zugeführt ist.

3. Anordnung nach Anspruch 1 oder 2, dadurch gekennzeichnet, daß eine Meßeinrichtung (5'') zur Messung der Beininnentemperatur, vorzugsweise an der Wade, vorgesehen ist.

4. Anordnung nach einem der Ansprüche 1 bis 3, dadurch gekennzeichnet, daß für die Person (1) ein thermisch charakterisierbarer Raum (2), z. B. ein kalorimeterähnlicher Behälter oder ein Inkubator, vorgesehen ist und daß zur Messung seiner Wand- und Außentemperatur die weiteren Meßfühler (3a, 3b) vorgesehen sind, deren Meßwerte mit dem Meßwert des Meßfühlers (3) für die Umgebungstemperatur in einer Schalteinheit (25) zur Ermittlung einer der wirksamen Umgebungstemperatur entsprechenden Signales verknüpfbar sind.

5. Anordnung nach einem der Ansprüche 1 bis 4, dadurch gekennzeichnet, daß die Auswerteeinheit (7) einen Speicher (9) für die Person (1) betreffende Daten, z. B. Gewicht, Länge, Volumen, Oberfläche des Rumpfes, Kopfes, der Arme und Beine und Rechenkonstante aufweist, wobei die Schalteinheiten der Auswerteeinrichtung (7) bzw. die in sie eingehenden Signalwerte mit den Speicherdaten verknüpfbar sind bzw. die einzelnen Schaltungen mit dem Speicher (9) verbunden sind.

6. Anordnung nach einem der Ansprüche 1 bis 5, dadurch gekennzeichnet, daß als Temperaturmeßfühler (4, 5) und als Meßfühler (3, 3a, 3b) Thermistoren vorgesehen sind.

## Claims

1. A system for measuring the heat dissipation and the skin temperature of a person to be examined, characterized in that

a) a temperature-measuring sensor (4) is adapted to be contacted with the torso of the person (1) to be examined and an optional heat flux sensor (4') are provided for respectively measuring the skin temperature on and the heat flux from a skin portion on the torso,

b) at least one additional temperature-measuring sensor (5) and an optional additional heat flux sensor (5') are provided and are adapted to be contacted with at least one additional part of the body, preferably a leg or an arm, and are respectively used to measure the skin temperature on and the heat flux from the respective skin portion,

c) if only the temperature-maasuring sensors (4, 5) are provided, at least one measuring sensor (3) is provided for a simultaneous and preferably continuous measurement of the ambient temperature and in combination with additional measuring sensors (3a, 3b) determines the effective ambient temperature,

d) the signal difference between the signal(s) representing the temperatures of the skin portion of the torso and the skin portion(s) of the other part(s) of the body and the signals representing the effective ambient temperature ist adapted to be determined in a subtracting circuit (10) of an

9

# 0 059 701

evaluating unit (7) from the signals of the temperature-measuring sensors (4, 5) and the signal representing the effective ambient temperature, and the output signal values of said subtracting circuit (10), as values which are proportional to the heat dissipation from the torso and from the respective part of the body, and optionally the output signal values of the heat flux sensors (4', 5') are delivered to a recording and/or indicating unit (8).

2. A system according to claim 1, characterized in that another temperature-measuring sensor (6) is provided for measuring the temperature inside the body and is adapted to be introduced, e.g., into the oesophagus, to the level of the heart, or into the anus, and that its output signal is delivered to the evaluating unit (7).

3. A system according to claim 1 or 2, characterized in that a measuring device (5'') is provided for measuring the temperature on the inside of a leg, preferably on the calf of the leg.

4. A system according to any of claims 1 to 3, characterized in that a thermally definable room (2), e.g., a calorimeterlike container or an incubator, is provided for the person (1), and that the additional measuring sensors (3a, 3b) are provided for measuring its wall and outside temperatures and their measured values are adapted to be combined in a circuitry unit (25) for the determination of a signal which corresponds to the effective ambient temperature with the measured value of the measuring sensor (3) for measuring the ambient temperature.

5. A system according to any of claims 1 to 4, characterized in that the evaluating unit (7) comprises a memory (9) for data relating to the person (1), such as weight, length, volume, surface area of torso, head, arms and leg, and constant operands, and the switching units of the evaluating device (7) or the signal values entered into the same are adapted to be combined with the data stored in the memory and/or the several circuits are connected to the memory (9).

6. A system according to any of claims 1 to 5, characterized in that thermistors are provided as temperature-measuring sensors (4, 5) and as measuring sensors (3, 3a, 3b).

## Revendications

1. Dispositif de mesure des données thermiques et de la température cutanée d'une personne à examiner, caractérisé en ce que

a) il est prévu un détecteur de température (4) à placer sur le torse de la personne à examiner (1) et éventuellement un tâteur de flux thermique (4') pour la mesure de la température cutanée et éventuellement du flux thermique d'un emplacement de peau sur le torse,

b) il est prévu au moins un autre détecteur de température (5) et éventuellement un autre tâteur de flux thermique (5') que l'on peut placer sur au moins une autre partie du corps, de préférence sur une jambe ou sur un bras, et qui mesure la température cutanée et le flux thermique à chaque emplacement de la peau,

c) il est prévu, pour une disposition du seul détecteur de température (4, 5) au moins un détecteur (3) pour la mesure simultanée et de préférence continue de la température environnante, qui, conjointement à d'autres détecteurs (3a, 3b), donne la température effective d'environnement,

d) les signaux du détecteur de température (4, 5) et du signal correspondant à la température effective d'environnement permettent de déterminer, dans un circuit soustracteur (10) d'une unité d'exploitation (7), la différence de signal entre les signaux correspondant à la température de chaque emplacement cutané du torse et du (ou des) emplacement (3) de peau de la ou des autres parties du corps et le signal correspondant à la température d'environnement efficace, les valuers de signal de sortie de ce circuit soustracteur (10) étant amenées comme valeurs proportionnelles aux données thermiques du torse et desdites parties du corps, ainsi qu'éventuellement les valeurs de signal de sortie du tâteur de flux thermique (4', 5') à une unité d'affichage ou d'enregistrement (8).

2. Dispositif selon la revendication 1, caractérisé en ce qu'il est prévu un autre détecteur de température (6) pour la mesure de l'intérieur du corps qui peut être introduit par exemple dans l'oesophage, au niveau du coeur ou dans l'anus, et en ce que son signal de départ est transmis à l'unité d'exploitation (7).

3. Dispositif selon la revendication 1 et la revendication 2, caractérisé en ce qu'il est prévu une installation de mesure (5'') pour la mesure de la température interne de la jambe, de préférence sur le mollet.

4. Dispositif selon l'une quelconque des revendications 1 à 3, caractérisé en ce qu'il est prévu pour la personne (1) un espace (2) caractérisable thermiquement, par exemple une chambre type calorimètre ou un incubateur, et en ce que sont prévus, pour la mesure de sa température de parois et de sa température extérieure, les autres détecteurs (3a, 3b), dont les valeurs de mesure sont combinables à la valeur de mesure du détecteur (3) pour la température d'environnement, dans une unité de distribution (25) pour la détermination d'un signal correspondant à la température effective de l'environnement.

5. Dispositif selon l'une des revendications 1 à 4, caractérisé en ce que l'unité d'exploitation (7) comporte une mémoire (9) pour les données concernant la personne, telles que le poids, la taille, le volume, la surface du torse, de la tête, du bras et de la jambe, ainsi que des constantes de calcul, l'unité de distribution de l'unité d'exploitation (7) et les valeurs de signaux qu'elle renferme étant couplables avec les données de la mémoire ou les connexions élémentaires étant reliées à la mémoire (9).

6. Dispositif selon l'une des revendications 1 à 5, caractérisé en ce que des thermistors sont prévus comme détecteurs de température (4, 5) et comme détecteurs (3, 3a, 3b).

Fig. 1

peripherer Blutfluß

Körper
Bein
} Hautblutfluß

Körper
Bein
} Gefäßwiderstand

gesamter Gefäßwiderstand

Körper
Bein
} Wärmeabgabe mit Temperaturmeßfühlern

Körper
Bein
} Hauttemperatur

Körper
Bein
} Wärmeabgabe mit heat flux disc

Beininnentemperatur

Körperkerntemperatur

Herzminutenvolumen

Körper
Bein
} Gewebeleitfähigkeit

Bein
Körper
} Wärmespeicherung

0 059 701

13

Fig. 3

Fig. 2